# EUROPEAN PATENT APPLICATION

(11) **EP 4 523 604 A1**
(43) Date of publication of application: **19.03.2025**
(21) Application number: 24200027.1
(22) Date of filing: 12.09.2024
(51) Int. Cl.: A61B 1/12, A61B 1/00, A61B 90/70

(54) **METHOD AND ARRANGEMENT FOR PRE-CLEANING AN ENDOSCOPE**

(30) Priority: 13.09.2023 US 202363538119 P
(71) Applicant: Olympus Winter & Ibe GmbH, 22045 Hamburg (DE)
(72) Inventor: Ottens, Benjamin, 22399 Hamburg (DE)
(74) Representative: Eisenführ Speiser

(57) **Abstract**

The invention relates to a method for pre-cleaning an endoscope. Further, the invention relates to an arrangement for pre-cleaning an endoscope. The method for pre-cleaning an endoscope is preferably conducted immediately or shortly after use of the endoscope and comprises: providing an endoscope that comprises channels for transporting fluids, connection ports, in particular for connection of fluid hoses, and a distal end of the endoscope, wherein each of the channels extends from one of the connection ports to the distal end of the endoscope; drawing liquid into at least one of the channels; determining an electrical parameter of fluid or fluids in at least one of the channels into which liquid has been drawn.

## Description

### TECHNICAL FIELD

The invention relates to a method for pre-cleaning an endoscope. Further, the invention relates to an arrangement for pre-cleaning an endoscope.

### BACKGROUND

An endoscope is an inspection instrument that can be used to examine, and optionally manipulate, the inside of organisms. Endoscopes can be used in human medical diagnostics for examinations or minimally invasive surgical procedures on humans or animals. Endoscopes typically comprise an image sensor, an optical lens, and a light source.

After use of an endoscope, the endoscope is typically pre-cleaned, manually or automated cleaned, disinfected, and possibly sterilized before being used again on a patient. If an endoscope and accessories used in the patient procedure are not immediately cleaned after each patient procedure, residual organic debris will begin to dry and solidify, hindering effective removal and reprocessing efficacy. Therefore, it is necessary to pre-clean the endoscope and the accessories at the bedside immediately after each patient procedure.

Typically, the pre-cleaning that is conducted at the bedside immediately after each patient procedure inter alia comprises the following steps: First, a biopsy/suction channel is pre-cleaned by arranging a distal end of the endoscope in water, and drawing the fluid through the endoscope. Then, air is drawn through the endoscope. Second, an air/water channel is pre-cleaned by arranging the distal end of the endoscope in water and flush the air/water channel with the fluid. Then, the air/water channel is flushed with air. Third, if available, an auxiliary water channel is pre-cleaned by flushing the auxiliary water channel with water. Finally, the accessories are detached from the endoscope and placed in a fluid, such as water or a detergent solution. Typically, after the pre-cleaning procedure the endoscope is detached from the equipment (such as for example video system center, light source, suction pump) used in the patient procedure and transported to a reprocessing area.

In US 11445900B2 it was suggested to pre-clean an endoscope by using a plurality of input members in form of flexible tubes for use in delivering cleaning solution to multiple input ports of an endoscope, so that a cleaning solution is delivered through the flexible tubes to the endoscope.

However, known procedures for pre-cleaning are both relatively time-consuming and prone to errors. As the pre-cleaning typically consists of several subsequently conducted manually applied steps, the pre-cleaning is relatively time-consuming and if only one of these steps is not conducted exactly according to the pre-cleaning procedure, the endoscope might not be pre-cleaned sufficiently. Furthermore, because not all endoscope channels are connected to each other it is not possible to flush all channels with only one source. In typically conducted pre-cleaning procedures, the video system center is required to flush the air/water channel, a suction pump to aspire the suction and biopsy channel and a flushing pump or syringe to flush the auxiliary water channel. Therefore, the user is permanently involved in the procedure and needs to interact with several different user interfaces which requires that the user can operate all of these user interfaces. Additionally a lot of equipment/accessories is necessary to conduct the pre-cleaning procedure.

Furthermore, a problem is that after pre-cleaning, drying and solidification can occur in the channels that are no longer filled with fluid.

Furthermore, in known procedures for pre-cleaning, there is typically a lack of verification possibilities to check whether the pre-cleaning was carried out properly and/or whether sufficient pre-cleaning has taken place.

In addition, there is typically a lack of possibilities to reliably document whether the pre-cleaning was carried out properly and/or whether sufficient pre-cleaning has taken place.

Furthermore, there is typically a lack of possibilities to check and/or verify whether liquid is present in the channels of the endoscope after pre-cleaning and/or whether liquid has been drawn out of the channels of the endoscope due to leakages.

Furthermore, there is typically a lack of possibility to identify blockages in the channels. Such blockages can lead to a situation in which liquid cannot be drawn through the channel in which a blockage is present. Such a blocked channel then does not get filled with liquid which can lead to an insufficiently pre-cleaning of the channels.

### SUMMARY

Therefore, it is an object of the present invention to provide an improved method and an improved arrangement for pre-cleaning an endoscope. In particular, it is an object of the present invention to provide a method for pre-cleaning an endoscope that is less prone to errors.

According to a first aspect, it is provided a method for pre-cleaning an endoscope, preferably immediately or shortly after use of the endoscope, the method comprising: providing an endoscope that comprises channels for transporting fluids, connection ports, in particular for connection of fluid hoses, and a distal end of the endoscope, wherein each of the channels extends from one of the connection ports to the distal end of the endoscope; drawing liquid into at least one of the channels; determining an electrical parameter of fluid or fluids in at least one of the channels into which liquid has been drawn.

In the following, initially the method for pre-cleaning an endoscope and its functions and advantageous embodiments will be described.

Preferably, the pre-cleaning procedure is conducted immediately or shortly after use of the endoscope in a patient procedure. Preferably the pre-cleaning procedure is started within 60 minutes after being used in the patient procedure, particularly preferably within 30 minutes after being used in the patient procedure, in particular within 15 minutes after being used in the patient procedure. Preferably, the pre-cleaning is conducted at a patient bedside so that the method can be conducted immediately or shortly after use of the endoscope in a patient procedure without the need to move the endoscope to another location prior to conducting the pre-cleaning.

Preferably, the channels each extend from one of the connection ports to the distal end of the endoscope. However, the endoscope can also have additional channels that do not extend from one of the connection ports to the distal end.

The distal end of the endoscope may also be referred to as the distal tip of the endoscope. Preferably, the distal end is to be understood as the end portion of the endoscope that is located at the very front of an insert section of the endoscope, in particular the end portion that is first inserted into the patient when the insert section of the endoscope is moved into a patient. Preferably, outlets of the channels are arranged at the distal end. Further preferably, a light guide is arranged at the distal end. Further preferably, an objective lens can be arranged at the distal end. It is particularly preferred if the distal end is arranged at the end of an insert section that has a flexible insertion tube.

Preferably, the connection ports are to be understood as ports to which fluid hoses or the like can be connected to.

Preferably, the endoscope can for example be a colonoscope, or a gastroscopes, or an ultrasonic endoscope, or a duodenoscopes, or another type of endoscope.

Preferably, drawing liquid into at least one of the channels is conducted by connecting a suction device to at least one of the connection ports and applying an underpressure by activating the suction device. Such a suction device can for example be a suction pump.

Preferably, determining an electrical parameter of fluid or fluids in at least one of the channels into which liquid has been drawn is conducted by measuring such an electrical parameter between two contact points that are preferably arranged at both ends, i.e. connection port and/or outlet opening, of a channel so that the corresponding channel substantially extends between these contact points.

Preferably, determining such an electrical parameter comprises measuring an electrical parameter. Then, for example, the electrical conductivity can be determined.

Preferably, the channel walls contain or consist of an electrical insulating material such as a polymer material that is substantially electrically insulating and has thus a relatively low electrical conductivity. Thus, the electrical conductivity measured along the extension of a channel is relatively low if the channel is filled with air due to the very low electrical conductivity of both the channel wall and the air inside of the channel. However, the electrical conductivity measured along the extension of a channel is significantly higher if the channel is filled with a liquid such as water or a disinfectant solution or a detergent solution. Therefore, the electrical conductivity and thus the electrical resistance can be very different depending on whether the channel is filled with air or a liquid.

An advantage of such a method is that by determining an electrical parameter it can reliably be determined whether the channel is filled with liquid or not. Therefore, it can be determined in a particularly advantageous way whether the pre-cleaning has been conducted sufficiently with respect to the corresponding channel and whether the channel is still filled with liquid if the measurement is conducted after flushing and/or drawing liquid through the corresponding channel.

Another advantage of such a method is that in a particularly advantageous way the patency of the channels can be checked. If there is a blockage in a channel and liquid cannot be drawn through the channel because of such a blockage, the channel does not get filled with liquid which results in a significantly lower electrical conductivity. This way it is possible to identify channel blockages and thus verify whether a patency of the corresponding channel is given. Thus, it is possible to proof the patency of the corresponding channel.

Another advantage of this method is that the channels can reliably be kept humid and/or filled with liquid after having conducted the pre-cleaning procedure. Thus, drying and solidification in the channels after pre-cleaning can be prevented in a reliably and particularly advantageous way.

In a particularly preferred embodiment, the method comprises: comparing the determined parameter of fluid or fluids with a predetermined threshold value; and preferably determining, in dependence of the comparison between the determined parameter of fluid or fluids and the threshold value, whether a liquid level and/or a humidity level in the channel is sufficient, and/or whether the channels are filled with liquid, and/or whether a patency of the channels is given, and/or whether an electrical measurement instrument for measuring the electrical parameter is electrically connected to the endoscope.

Preferably, the determined electrical parameter is compared to the predetermined threshold value, wherein depending on whether the determined electrical parameter lies above or below the threshold value, it is determined whether the corresponding channel is filled with liquid.

An advantage of such a comparison is that it can reliably be determined whether the corresponding channel is filled with liquid and/or whether patency of the corresponding channel is given. By using such a predetermined threshold value, it is easy for the user to determine whether the desired liquid level and/or humidity level in the channel is reached and whether the patency of the channel is given by just comparing the measured value to the predetermined threshold.

Another advantage of such a comparison with a predetermined value is that the comparison can be conducted automatically, for example by using a processing unit that is adapted to compare the determined parameter with the predetermined threshold value and preferably dependent on the comparison output data comprising information on whether the determined value lies above or below the threshold and/or whether the corresponding is sufficiently filled with liquid and/or whether patency of the corresponding channel is given.

Another advantage of such a comparison is that it can reliably be determined whether the measurement instrument for measuring the electrical parameter is electrically connected to the endoscope in a correct manner.

In a further preferred embodiment, the method comprises: fluidly connecting the channels to each other at the distal end of the endoscope by arranging a container onto the distal end of the endoscope; connecting a suction device, preferably a suction pump, to one of the connection ports; connecting at least one liquid source to at least one of the connection ports; drawing the liquid into at least one of the channels by activating the suction device so that the liquid is drawn from the liquid source through the at least one channel via the distal end towards the suction device.

Preferably, at least two of the channels are flushed with a liquid by activating the suction device so that the liquid is drawn from the liquid source through at least one of the channels via the distal end and then from the distal end through at least one other channel of the channels towards the suction device.

Preferably, the container can be arranged onto the distal end in a condition in which it is filled with liquid or in which it is not filled with liquid. If the container is not filled with liquid, a continuous flow of liquid can be drawn through the channels via the distal end.

Preferably, before flushing channels of the endoscope, the container is not filled with liquid. An advantage of such an empty container is that liquid can be drawn from a connection port through a channel to the container and from the container through a suction channel towards the suction device out of the endoscope, wherein no gaps of liquid that contain gas are present. Thus, when liquid is drawn out of a connection port, then the user can be sure that this liquid has been drawn completely through at least two channels. However, alternatively, it is also possible that the container is already filled with liquid before flushing the channels. Filling the container with liquid before starting the flushing of the channels can have the advantage that the distal end of the endoscope is in contact with liquid for a longer period of time and soaked for a longer period of time, which can be beneficial to achieve a more thorough pre-cleaning of the distal end, if needed.

An advantage of arranging such a container onto the distal end of the endoscope is that even though not all channels of the endoscope are connected to each other inside of the endoscope, it is possible to flush all of these channels with only one source by connecting the cannels to each other via the container that is arranged onto the distal end of the endoscope. Therefore, the pre-cleaning procedure can be conducted faster, easier and in a way that is less prone to errors.

Another advantage of arranging such a container onto the distal end of the endoscope is that during conducting the pre-cleaning of the endoscope it is not necessary to interact with several user interfaces and/or devices in order to conduct the pre-cleaning procedure.

Another advantage of arranging such a container onto the distal end of the endoscope is that compared to conventional pre-cleaning methods, with the described method, much less equipment and/or accessories is necessary to conduct the pre-cleaning. For example, no video center or flushing pump is necessary to conduct the pre-cleaning.

In a further preferred embodiment, the electrical parameter is determined by electrical resistance measurement and/or electrical voltage measurement and/or electrical current measurement and/or electrical conductance measurement, preferably by using an ohmmeter and/or a multimeter.

Preferably, the electrical parameter is determined by measurement of the electrical resistance and/or the electrical voltage and/or the electrical current and/or the electrical conductance, and optionally by calculating the electrical parameter from the measured values.

In a further preferred embodiment, the electrical resistance measurement and/or electrical voltage measurement and/or electrical current measurement and/or electrical conductance measurement comprises contacting the channel in which the electrical parameter of fluid or fluids is to be determined.

Preferably, the channel or the channels in which the electrical parameter of fluid or fluids is to be determined are electrically contacted at the ends of the corresponding channel or channels so that, using an electrical measurement instrument, the electrical parameter can be determined for the corresponding channel or channels.

In a further preferred embodiment, determining an electrical parameter of the fluid or fluids in at least one of the channels comprises determining an electrical resistance between two distanced points of the channels or an electrical resistivity; and/or determining an electrical conductance between two distanced points of the channels or an electrical conductivity.

Preferably, the electrical resistance and/or electrical conductance is determined between two ends of a channel.

In a further preferred embodiment, one of the two distanced points between which an electrical measurement is conducted is positioned at or adjacent to the connection port of the corresponding channel and/or one of the two distanced points between which an electrical measurement is conducted is positioned at or adjacent to the connection port to which the suction device is connected.

Preferably, when a container is arranged at the distal end of the endoscope, the electrical measurement is conducted between a first connection point that is arranged at a connection port of a first channel and the connection port of the suction channel. This way the whole extension of the first channel and the whole extension of the suction channel can be electrically measured as these channels are fluidly connected to each other via the container that is arranged at the distal end of the endoscope.

In a further preferred embodiment, drawing liquid into at least one of the channels comprises filling the corresponding channel completely with the liquid.

Preferably, after drawing liquid into the channels, the liquid is kept in the channels to avoid solidification of residual organic debris.

In a further preferred embodiment, connecting at least one liquid source to at least one of the connection ports comprises connecting at least one vessel that holds liquid, preferably at least three, in particular four, vessels that hold liquid, to at least one of the connection ports, preferably to at least three, in particular four, of the connection ports.

An advantage of connecting at least one vessel that holds liquid to at least one of the connection ports is that, in particular by applying an underpressure, the liquid can be drawn from the vessel through the channel that extends from the connection port to which the vessel is connected to the distal end of the endoscope and when a container is arranged at the distal end the liquid can further be drawn from the distal end through another channel, in particular towards a suction device. This way it can be achieved that the liquid is drawn through the entire extension of both of these channels starting from the vessel, through the first channel via the distal end and then through the other channel, so that both of these channels are reliably flushed with the liquid along their whole extension. Further channels can be reliably flushed in the same way. Therefore, it is preferable to arrange vessels onto several connection ports so that all of the channels that extend from these connection ports to the distal end can reliably be flushed with liquid along their whole extension.

In a further preferred embodiment, the channels comprise an air channel that extends from an air channel connection port to the distal end of the endoscope, a suction channel that extends from a suction channel connection port to the distal end of the endoscope, a water channel that extends from a water channel connection port to the distal end of the endoscope, and preferably an auxiliary water channel that extends from an auxiliary water channel connection port to the distal end of the endoscope.

Preferably, the air channel is adapted to provide air to the distal end of the endoscope. Preferably, the suction channel is adapted to draw liquids and/or objects, such as for example tissue samples, from the distal end of the endoscope through the endoscope. Preferably, the water channel is adapted to provide water at the distal end of the endoscope. Preferably, the auxiliary water channel is adapted to provide water at the distal end of the endoscope.

Preferably, the channels are arranged parallel to each other over at least a part of their extension from one of the connection ports to the distal end of the endoscope.

In a further preferred embodiment, the method comprises: providing an RFID transponder that contains information about at least one pre-cleaning parameter, wherein preferably the at least one pre-cleaning parameter comprises at least one dynamic parameter that comprises information about the determined electrical parameter of fluid or fluids in at least one of the channels into which liquid has been drawn and/or information about the humidity in at least one of the channels of the endoscope.

RFID is in particular to be understood as Radio-frequency identification (RFID). RFID can in particular use electromagnetic fields to automatically identify and track tags.

An RFID transponder preferably comprises an antenna, an electrical circuit for receiving and transmitting data, and a digital circuit with memory. The antenna, the electrical circuit and the digital circuit can be integrated in a single microchip.

Preferably, the RFID transponder comprises a memory that can be written to at least once and comprises an unchangeable identity.

Preferably the at least one pre-cleaning parameter comprises at least one static parameter that comprises information about an expiry date of the liquid to be drawn into at least one of the channels. Preferably, the at least one static parameter comprises information on a reference number to prove the use of a correct product and/or information on the expiry date to prove the validity of the expiry date.

Preferably the at least one dynamic parameter can be an electrical resistance and/or an electrical voltage and/or an electrical current and/or an electrical conductance. Preferably, the at least one dynamic parameter can comprise information about whether an electrical parameter lies above a predetermined threshold value. Preferably, the at least one dynamic parameter can comprise information on whether a liquid level and/or a humidity level in the channel is sufficient, and/or whether the channels are filled with liquid, and/or whether a patency of the channels is given, and/or whether an electrical measurement instrument for measuring the electrical parameter is electrically connected to the endoscope.

Preferably, the dynamic parameter can in particular comprise a measured value or measured values, in particular in form of raw data, that can be interpreted after being read out by an RFID reader. Such an RFID reader can for example be arranged external of the RFID circuit.

Preferably, information on electrical resistance and/or an electrical voltage and/or an electrical current and/or an electrical conductance and/or further parameters can be written to the memory of the RFID transponder, wherein further parameters can for example be temperature, and/or pressure values at the time of conducting the pre-cleaning procedure.

In a further preferred embodiment, the at least one pre-cleaning parameter is determined by using a measurement instrument, preferably an electrical measurement instrument, wherein the RFID transponder, in particular an RFID antenna of the RFID transponder, provides power to operate the measurement instrument.

Such an electrical measurement instrument can in particular be an ohmmeter and/or a multimeter. Preferably, the energy needed for measuring and/or determining the at least one pre-cleaning parameters can be provided via the RFID antenna of the RFID transponder.

An advantage of providing energy of the RFID transponder for conducting measurements needed to determine at least one pre-cleaning parameter is that no additional battery or any other portable energy source is needed to provide energy for such conducting measurements.

In a further preferred embodiment, the RFID transponder is arranged on a portable, and preferably disposable, device. Preferably an RFID antenna is arranged on the portable device.

An advantage of using such a portable and disposable device is that a disposable ready-to-use solution for pre-cleaning can be provided that is easy to handle by a user and can easily be disposed after use.

In a further preferred embodiment, the method comprises: reading out information stored on the RFID transponder and in dependence of these information continuing with reprocessing of the endoscope or conducting further cleaning steps for cleaning the endoscope, in particular for cleaning the channels of the endoscope.

Preferably, after reading out information stored on the RFID transponder, it can be determined whether a sufficient pre-cleaning was conducted and/or whether channels that should be filled with liquid are filled with liquid. Thus, based on the information read out after conducting the pre-cleaning procedure, it can be decided, whether the pre-cleaning has been conducted in a correct way and/or whether further pre-cleaning steps have to be conducted to make sure that a sufficient pre-cleaning is conducted.

In a further preferred embodiment, the method comprises: after pre-cleaning, reading out the RFID transponder, preferably by using an RFID reader, preferably at a reprocessing location, in particular prior to reprocessing of the endoscope.

This way it is possible to check and/or verify and/or document the data that has been written onto the RFID transponder before conducting further reprocessing steps.

Preferably, after reading out the RFID transponder, it can be checked whether the parameters are valid and whether the pre-cleaning has been conducted sufficiently. In dependence of this data it can then be decided whether the reprocessing can be started or whether additional cleanings steps, such as for example brushing and/or flushing and/or immersing are necessary before continuing the reprocessing of the endoscope.

The advantages explained above with respect to the method for pre-cleaning are also advantages for such an arrangement for pre-cleaning.

According to a further aspect, it is provided an arrangement for pre-cleaning an endoscope, preferably immediately or shortly after use of the endoscope, in particular according to a method as described herein, the arrangement comprising: an endoscope that comprises channels for transporting fluids, connection ports, in particular for connection of fluid hoses, and a distal end of the endoscope, wherein each of the channels extends from one of the connection ports to the distal end of the endoscope; a device, in particular an electrical measurement instrument, for determining an electrical parameter of fluid or fluids in at least one of the channels of the endoscope.

As to the advantages, preferred embodiments and details of the individual different aspects and their preferred embodiments, reference is also made to the corresponding advantages, preferred embodiments and details described with reference to the respective other aspects.

Further advantageous embodiments result from the combination of individual, several or all of the preferred features described herein.

### BRIEF DESCRIPTION OF THE DRAWINGS

Preferred embodiments shall now be described with reference to the attached drawings, in which
- Fig. 1:: shows a schematic depiction of an exemplary embodiment of an arrangement for pre-cleaning an endoscope;
- Fig. 2:: shows a schematic arrangement of an exemplary embodiment for determining an electrical parameter;
- Fig. 3:: shows a schematic arrangement of an exemplary embodiment for determining an electrical parameter;
- Fig. 4:: shows a schematic flow diagram of an exemplary embodiment of a method for pre-cleaning an endoscope;
- Fig. 5:: shows a schematic flow diagram of an exemplary embodiment of a method for pre-cleaning an endoscope.

### DETAILED DESCRIPTION

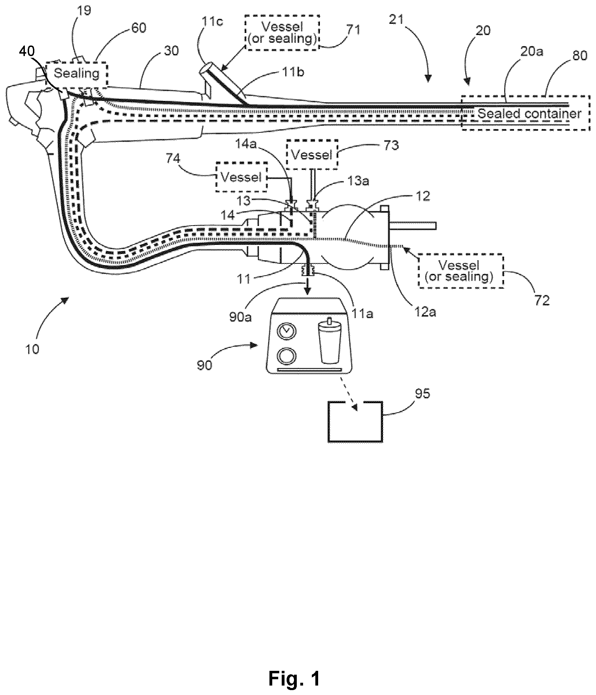

Fig. 1 shows a schematic depiction of an exemplary embodiment of an arrangement for pre-cleaning an endoscope 10. The endoscope 10 can be pre-cleaned immediately or shortly after use of the endoscope 10. The endoscope 10 comprises an endoscope body 30 and channels 11, 12, 13, 14 for transporting fluids, namely a suction channel 11, an air channel 12, a water channel 13, and an auxiliary water channel 14.

The suction channel 11 is adapted to draw liquids and/or objects, such as for example a tissue sample, from the distal end 20 of the endoscope 10 through the endoscope 10. The suction channel 11 extends from a suction channel connection port 11a to the distal end 20 of the endoscope 10. The distal end 20 is the end portion of the endoscope 10 that is located at the very front of an insert section 21 of the endoscope 20.

The air channel 12 is adapted to provide air to the distal end 20 of the endoscope 10. The air channel 12 extends from an air channel connection port 12a to the distal end 20 of the endoscope 10.

The water channel 13 is adapted to provide water at the distal end 20 of the endoscope 10. The water channel 13 extends from a water channel connection port 13a to the distal end 20 of the endoscope 10.

The auxiliary water channel 14 is adapted to provide water at the distal end 20 of the endoscope 10. The auxiliary water channel 14 extends from an auxiliary water channel connection port 14a to the distal end 20 of the endoscope 10.

The connection ports 11a, 11c, 12a, 13a, 14a are adapted for connection of fluid hoses and/or vessels and/or closure elements. Each of the channels 11, 12, 13, 14 extends from one of the connection ports 11a, 12a, 13a, 14a to the distal end 20 of the endoscope 10.

The channels 11, 12, 13, 14 are fluidly connected to each other at the distal end 20 of the endoscope 10 by arranging a container 80 onto the distal end 20 of the endoscope 10. The container 80 is arranged onto the distal end 20 of the endoscope 10 in such a manner that the distal end 20, including the outer circumferential surface 20a of the distal end 20, is arranged entirely inside the container 80. If the container 80 gets filled with liquid, the whole distal end 20, including the outer surface 20a of the distal end 20, is in contact with the liquid so that a reliable pre-cleaning of the whole distal end 20 can be achieved. The container 80 is arranged on the distal end 20 of the endoscope 10 in such a manner that the distal end 20 of the endoscope 10 is sealed liquid-tight and gas-tight from the environment that surrounds the container 80.

The endoscope 10 furthermore comprises a biopsy branch 11b that extends from a biopsy branch connection port 11c to the suction channel 11.

The air channel 12 and the water channel 13 can be fluidly connected and/or separated from each other via an air/water cylinder 19. A vent can be assembled to the air/water cylinder 19. The air/water cylinder 19 is sealed with a closure element 60 that is adapted to close the air/water cylinder 19 fluid-tight. Furthermore, a suction cylinder 40 is provided to which the suction channel 11 is connected to. A vent can be assembled to the suction cylinder 40.

A vessel 71 is arranged onto the biopsy branch connection port 11c. If the biopsy branch 11b does not need to be flushed during the pre-cleaning procedure, a closure element can be arranged onto the biopsy branch connection port 11c instead of the vessel 71.

A vessel 72 is arranged onto the connection port 12a of the air channel 12. If the air channel 12 does not need to be flushed during the pre-cleaning procedure, a closure element can be arranged onto the connection port 12a instead of the vessel 72.

A vessel 73 is arranged onto the connection port 13a of the water channel 13. If the water channel 13 does not need to be flushed during the pre-cleaning procedure, a closure element can be arranged onto the connection port 13a instead of the vessel 73.

Furthermore, a vessel 74 is arranged onto the connection port 14a of the auxiliary water channel 14. If auxiliary water channel 14 does not need to be flushed during the pre-cleaning procedure, a closure element can be arranged onto the connection port 14a instead of the vessel 74.

The vessels 71, 72, 73, 74 are filled with liquid, in particular with a disinfectant solution and/or detergent solution.

A suction device 90 that is a suction pump in this preferred embodiment is connected via a fluid hose 90a to the connection port 11a of the suction channel 11. When the suction device 90 is activated, an underpressure is applied to the system so that liquid is drawn towards the suction device 90 as described in detail below.

Liquid is drawn from the vessel 71 through the biopsy branch 11b and from there further through the part of the suction channel 11 that extends from the connection to the biopsy branch 11b and the distal end 20 towards the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

Liquid is drawn from the vessel 72 through the air channel 12 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

Liquid is drawn from the vessel 73 through the water channel 13 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

Liquid is drawn from the vessel 74 through the auxiliary water channel 14 to the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

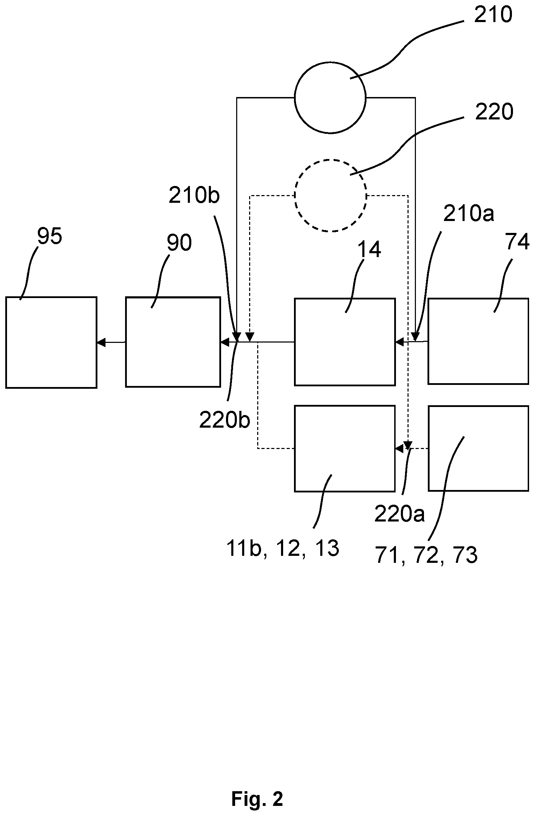

Fig. 2 shows a schematic arrangement of an exemplary embodiment for determining an electrical parameter. The schematic arrangement shown here can for example be applied to an arrangement and/or an endoscope as shown in Fig. 1. However, the schematic arrangement shown here can also be applied to other arrangements, for example used in connection with an endoscope as shown in Fig. 1 with or without a container arranged at the distal end of the endoscope or in connection with an endoscope similar to the one shown in Fig. 1 with or without a container arranged at the distal end of the endoscope.

A pre-cleaning procedure can be conducted as follows: An endoscope to be pre-cleaned is provided. A channel 14, for example an auxiliary water channel 14, but could be any other channel of the endoscope, has a connection port to which a liquid source in form of a vessel 74 is connected and to which on the other end of the channel 14 a suction device 90 is connected. Furthermore, additional channels 11b, 12, 13 can also be connected to vessels 71, 72, 73 at their connection ports and to the suction device 90 in a direct or indirect way if liquid shall also be drawn into these additional channels. The suction device 90 is further connected to a suction container, formed as a suction jar 95. Vessels 74, 71, 72, 73 that contain liquid are arranged onto the connection ports of the channels 11b, 12, 13, 14. By activating the suction device 90, liquid is drawn in the direction indicated by the arrows, namely from the vessels 74, 71, 72, 73 through the channels 14, 11b, 12, 13 (to which the vessels 74, 71, 72, 73 are connected to) towards the suction pump 90 to be then collected in the suction jar 95.

An electrical measurement instrument 210, for example an ohmmeter or multimeter, is connected to the channel 14 at two distanced contact points 210a, 210b. If further channels 11b, 12, 13 need to be pre-cleaned at least one additional electrical measurement instrument 220 can be used for measurements of the further channels 11b, 12, 13 by connecting the instrument to contact points 220a, 220b of each of the channels. However, it is also possible to measure all channels of interest, for example channels 14, 11b, 12, 13 with only one electrical measurement instrument. In this case reference numbers 210 and 220 belong to only one, i.e. to the same, electrical measurement instrument.

The electrical measurement instrument 210, 220 can conduct an electrical measurement to determine, based on this measurement, an electrical parameter of fluid or fluids in channel 14 and possibly in channels 11b, 12, 13, into which liquid has been drawn. Then the determined parameter of fluid or fluids can be compared with a predetermined threshold value and it can be determined, in dependence of the comparison between the determined parameter of fluid or fluids and the threshold value, whether a liquid level and/or a humidity level in the channel is sufficient, and/or whether the channels are filled with liquid, and/or whether a patency of the channels is given, and/or whether the electrical measurement instrument for measuring the electrical parameter is correctly electrically connected to the endoscope.

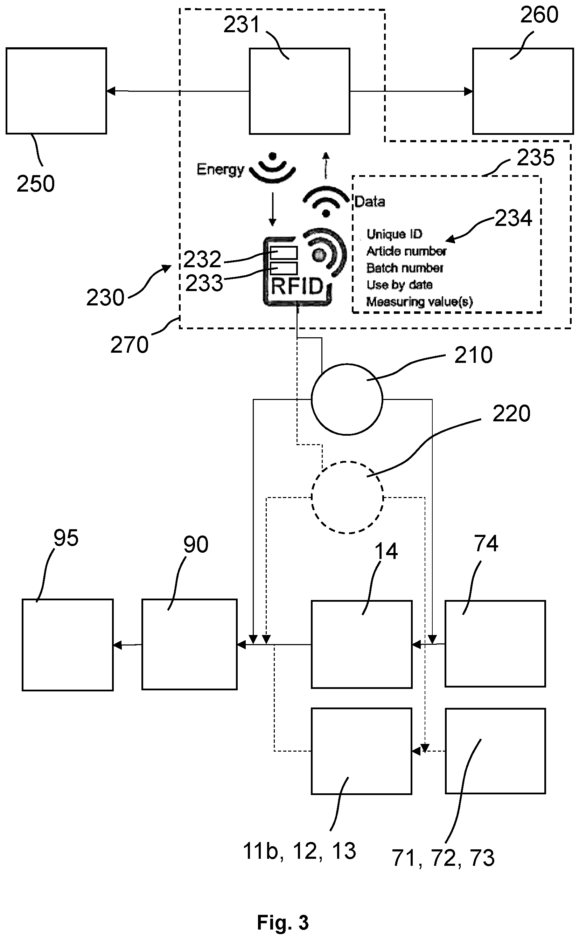

Fig. 3 shows a schematic arrangement of an exemplary embodiment for determining an electrical parameter. The embodiment shown here is the same as described in connection with Fig. 2, wherein additionally, an RFID transponder 230 with an RFID antenna 231 and an Extended Warehouse Management (EWM) software 250 and an endoscope reprocessor 260 are provided. The RFID transponder 230 contains information about at least one pre-cleaning parameter 234, wherein preferably the at least one pre-cleaning parameter 234 comprises at least one dynamic parameter that comprises information about the determined electrical parameter of fluid or fluids in at least one of the channels 14, 11b, 12, 13 into which liquid has been drawn and/or information about the humidity in at least one of the channels 11b, 12, 13 of the endoscope. The RFID transponder 230 comprises an RFID antenna 231, an electrical circuit 232 for receiving and transmitting data, and a digital circuit 233 with a memory. The antenna 231, the electrical circuit 232 and the digital circuit 233 can be integrated in a single microchip. The RFID transponder comprises a memory 235 that can be written to at least once and comprises an unchangeable identity.

The pre-cleaning parameters 234 comprise at least one static parameter that comprises information about an expiry date of the liquid to be drawn into at least one of the channels. Preferably, the at least one static parameter comprises information on a reference number to prove the use of a correct product and/or information on the expiry date to prove the validity of the expiry date.

The at least one dynamic parameter can in particular be an electrical resistance and/or an electrical voltage and/or an electrical current and/or an electrical conductance. The dynamic parameter can in addition or alternatively comprise information about whether an electrical parameter lies above a predetermined threshold value. In particular, the at least one dynamic parameter can comprise information on whether a liquid level and/or a humidity level in the channel is sufficient, and/or whether the channels are filled with liquid, and/or whether a patency of the channels is given, and/or whether an electrical measurement instrument for measuring the electrical parameter is electrically connected to the endoscope.

The RFID antenna 231 of the RFID transponder 230, provides power to operate the electrical measurement instrument 210, 220. Thus, no additional battery or any other portable energy source is needed to provide energy for conducting the electrical measurements.

The RFID transponder 230 is arranged on a portable and disposable device 270.

The RFID transponder 230 can be read out and data can be transmitted to the EWM software 250. Furthermore, the RFID transponder 230 can be read out by an endoscope reprocessor 260 in order to decide in dependence of these information whether it can be continued with a reprocessing of the endoscope or whether further cleaning steps for cleaning the endoscope, in particular for cleaning the channels of the endoscope, need to be conducted before. After reading out the RFID transponder 230, it can in particular be checked whether the parameters are valid and whether the pre-cleaning has been conducted sufficiently. In dependence of this data it can then be decided whether the reprocessing can be started or whether additional cleanings steps, such as for example brushing and/or flushing and/or immersing are necessary before continuing the reprocessing of the endoscope.

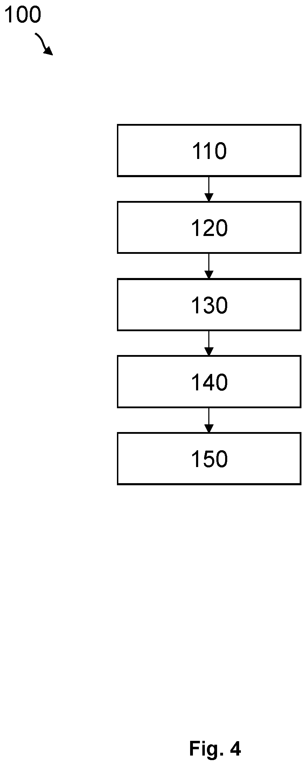

Fig. 4 shows a schematic flow diagram of an exemplary embodiment of a method 100 for pre-cleaning an endoscope, for example an endoscope as shown and described in connection with Fig. 1. The method 100 is conducted immediately or shortly after use of the endoscope in a patient procedure and comprises the following steps:

In a step 110, providing an endoscope that comprises channels for transporting fluids, connection ports, in particular for connection of fluid hoses, and a distal end of the endoscope, wherein each of the channels extends from one of the connection ports to the distal end of the endoscope

In a step 120, drawing liquid into at least one of the channels.

In a step 130, determining an electrical parameter of fluid or fluids in at least one of the channels into which liquid has been drawn, comprising determining an electrical resistance between two distanced points of the channels or an electrical resistivity and/or determining an electrical conductance between two distanced points of the channels or an electrical conductivity.

In a step 140, comparing the determined parameter of fluid or fluids with a predetermined threshold value.

In a step 150, determining, in dependence of the comparison between the determined parameter of fluid or fluids and the threshold value, whether a liquid level and/or a humidity level in the channel is sufficient, and/or whether the channels are filled with liquid, and/or whether a patency of the channels is given, and/or whether an electrical measurement instrument for measuring the electrical parameter is electrically connected to the endoscope.

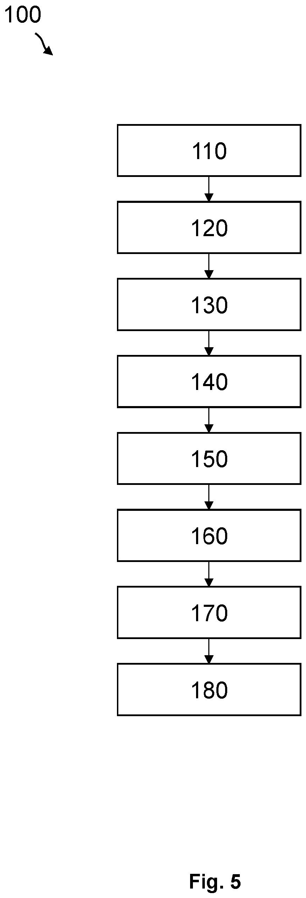

Fig. 5 shows a schematic flow diagram of an exemplary embodiment of a method 100 for pre-cleaning an endoscope, for example an endoscope as shown and described in connection with Fig. 1. The method described in connection with Fig. 5 comprises the same method steps 110, 120, 130, 140, 150 as described in connection with Fig. 4. In addition to these method steps, the method comprises the following steps:

In a step 160, providing an RFID transponder that contains information about at least one pre-cleaning parameter, wherein the at least one pre-cleaning parameter comprises at least one dynamic parameter that comprises information about the determined electrical parameter of fluid or fluids in at least one of the channels into which liquid has been drawn and/or information about the humidity in at least one of the channels of the endoscope, wherein the at least one pre-cleaning parameter comprises at least one static parameter that comprises information about an expiry date of the liquid to be drawn into at least one of the channels, wherein the at least one pre-cleaning parameter is determined by using a measurement instrument, preferably an electrical measurement instrument, wherein the RFID transponder, in particular an RFID antenna of the RFID transponder, provides power to operate the measurement instrument. The RFID transponder is arranged on a portable and disposable device.

In a step 170, reading out information stored on the RFID transponder and in dependence of these information continuing with reprocessing of the endoscope or conducting further cleaning steps for cleaning the endoscope, in particular for cleaning the channels of the endoscope.

In a step 180, after pre-cleaning, reading out the RFID transponder, preferably by using an RFID reader, preferably at a reprocessing location, in particular prior to reprocessing of the endoscope.

## Claims

1. A method for pre-cleaning an endoscope, preferably immediately or shortly after use of the endoscope, the method comprising:
- providing an endoscope that comprises
∘ channels for transporting fluids,
∘ connection ports, in particular for connection of fluid hoses, ∘ and a distal end of the endoscope,
wherein each of the channels extends from one of the connection ports to the distal end of the endoscope;
- drawing liquid into at least one of the channels;
- determining an electrical parameter of fluid or fluids in at least one of the channels into which liquid has been drawn.

2. The method according to the preceding claim, comprising:
- comparing the determined parameter of fluid or fluids with a predetermined threshold value;
- and preferably determining, in dependence of the comparison between the determined parameter of fluid or fluids and the threshold value,
∘ whether a liquid level and/or a humidity level in the channel is sufficient, and/or
∘ whether the channels are filled with liquid, and/or
∘ whether a patency of the channels is given, and/or
∘ whether an electrical measurement instrument for measuring the electrical parameter is electrically connected to the endoscope.

3. The method according to at least one of the preceding claims, comprising:
- fluidly connecting the channels to each other at the distal end of the endoscope by arranging a container onto the distal end of the endoscope;
- connecting a suction device, preferably a suction pump, to one of the connection ports;
- connecting at least one liquid source to at least one of the connection ports;
- drawing the liquid into at least one of the channels by activating the suction device so that the liquid is drawn from the liquid source through the at least one channel via the distal end towards the suction device.

4. The method according to at least one of the preceding claims,
wherein the electrical parameter is determined by electrical resistance measurement and/or electrical voltage measurement and/or electrical current measurement and/or electrical conductance measurement, preferably by using an ohmmeter and/or a multimeter.

5. The method according to the preceding claim,
wherein the electrical resistance measurement and/or electrical voltage measurement and/or electrical current measurement and/or electrical conductance measurement comprises contacting the channel in which the electrical parameter of fluid or fluids is to be determined.

6. The method according to at least one of the preceding claims,
wherein determining an electrical parameter of the fluid or fluids in at least one of the channels comprises
- determining an electrical resistance between two distanced points of the channels or an electrical resistivity;
- and/or determining an electrical conductance between two distanced points of the channels or an electrical conductivity,
wherein preferably one of the two distanced points between which an electrical measurement is conducted is positioned at or adjacent to the connection port of the corresponding channel and/or preferably one of the two distanced points between which an electrical measurement is conducted is positioned at or adjacent to the connection port to which the suction device is connected.

7. The method according to at least one of the preceding claims,
wherein drawing liquid into at least one of the channels comprises filling the corresponding channel completely with the liquid.

8. The method according to at least one of the preceding claims,
wherein connecting at least one liquid source to at least one of the connection ports comprises connecting at least one vessel that holds liquid, preferably at least three, in particular four, vessels that hold liquid, to at least one of the connection ports, preferably to at least three, in particular four, of the connection ports.

9. The method according to at least one of the preceding claims,
wherein the channels comprise
- an air channel that extends from an air channel connection port to the distal end of the endoscope,
- a suction channel that extends from a suction channel connection port to the distal end of the endoscope,
- a water channel that extends from a water channel connection port to the distal end of the endoscope,
- and preferably an auxiliary water channel that extends from an auxiliary water channel connection port to the distal end of the endoscope.

10. The method according to at least one of the preceding claims, comprising:
- providing an RFID transponder that contains information about at least one pre-cleaning parameter,
wherein preferably the at least one pre-cleaning parameter comprises at least one dynamic parameter that comprises information about the determined electrical parameter of fluid or fluids in at least one of the channels into which liquid has been drawn and/or information about the humidity in at least one of the channels of the endoscope,
wherein preferably the at least one pre-cleaning parameter comprises at least one static parameter that comprises information about an expiry date of the liquid to be drawn into at least one of the channels.

11. The method according to the preceding claim,
wherein the at least one pre-cleaning parameter is determined by using a measurement instrument, preferably an electrical measurement instrument, wherein the RFID transponder, in particular an RFID antenna of the RFID transponder, provides power to operate the measurement instrument.

12. The method according to at least one of the preceding claims,
wherein the RFID transponder is arranged on a portable, and preferably disposable, device,
wherein preferably an RFID antenna is arranged on the portable device.

13. The method according to at least one of the preceding claims, comprising:
- reading out information stored on the RFID transponder and in dependence of these information continuing with reprocessing of the endoscope or conducting further cleaning steps for cleaning the endoscope, in particular for cleaning the channels of the endoscope.

14. The method according to at least one of the preceding claims, comprising:
- after pre-cleaning, reading out the RFID transponder, preferably by using an RFID reader, preferably at a reprocessing location, in particular prior to reprocessing of the endoscope.

15. An Arrangement for pre-cleaning an endoscope, preferably immediately or shortly after use of the endoscope, in particular according to a method according to at least one of the preceding claims, the arrangement comprising:
- an endoscope that comprises
∘ channels for transporting fluids,
∘ connection ports, in particular for connection of fluid hoses, ∘ and a distal end of the endoscope,
wherein each of the channels extends from one of the connection ports to the distal end of the endoscope;
- a device, in particular an electrical measurement instrument, for determining an electrical parameter of fluid or fluids in at least one of the channels of the endoscope.
